# FASCICULE DE BREVET EUROPEEN

(11) **EP 2 488 484 B1**
(45) Date de publication et mention de la délivrance du brevet: **07.12.2016**
(21) Numéro de dépôt: 10785118.0
(22) Date de dépôt: 15.10.2010
(51) Int. Cl.: C07C 247/22, C08G 71/04

(54) **SYNTHÈSE DE POLYURÉTHANE PAR AUTOCONDENSATION**
POLYURETHANSYNTHESE DURCH SELBSTKONDENSATION
POLYURETHANE SYNTHESIS BY MEANS OF SELF-CONDENSATION

(30) Priorité: 15.10.2009 FR 0957223
(43) Date de publication de la demande: 22.08.2012
(73) Titulaire: Centre National de la Recherche Scientifique (C.N.R.S.), 75016 Paris (FR)
(72) Inventeur: CRAMAIL, Henri, F-33350 Sainte-Terre (FR); BOYER, Aurélie, F-33000 Bordeaux (FR); PALASKAR, Dnyaneshwar, IN-Bangalore 560081 (IN); CLOUTET, Eric, F-33880 Saint Caprais de Bordeaux (FR); ALFOS, Carine, F-33600 Pessac (FR)
(74) Mandataire: Lavoix
(86) Numéro de dépôt international: PCT/FR2010/052190
(87) Numéro de publication internationale: WO 2011/045546

(56) Documents cités:
- US-A1- 2003 065 188
- DATABASE BEILSTEIN [Online] BEILSTEIN INSTITUTE FOR ORGANIC CHEMISTRY, FRANKFURT-MAIN, DE; 3 avril 2003 (2003-04-03), k.l.Widdowson et al.: "2-Azidocarbonylpentane", XP002581760, Database accession no. 3030004
- KUMAR A ET AL: "A Novel One-pot Synthesis of Hyperbranched Polyurethanes", JOURNAL OF THE CHEMICAL SOCIETY, CHEMICAL COMMUNICATIONS, CHEMICAL SOCIETY. LETCHWORTH, GB LNKD- DOI:10.1039/C39930001453, 1 janvier 1993 (1993-01-01), pages 1453-1454, XP000650746, ISSN: 0022-4936 cité dans la demande
- ANIL KUMAR ET AL: "HYPERBRANCHED POLYURETHANES WITH VARYING SPACER SEGMENTS BETWEEN THE BRANCHING POINTS", JOURNAL OF POLYMER SCIENCE, POLYMER CHEMISTRY EDITION, INTERSCIENCE PUBLISHERS, NEW YORK, NY, US, vol. 34, no. 5, 15 avril 1996 (1996-04-15) , pages 839-848, XP000556930, ISSN: 0360-6376 cité dans la demande
- A. KUMAR ET AL.: "Synthesis of new thermotropic liquid crystalline polyurethanes containing biphenyl mesogens using a novel AB-type self-polycondensation.", CHEMICAL COMMUNICATION, 24 novembre 2003 (2003-11-24), pages 154-155, XP002581761, cité dans la demande
- N.D. GHATGE ET AL.: "Synthesis, characterization and properties of novel poly(ether Urethanes)", JOURNAL OF POLYMER SCIENCE, vol. 21, no. 7, 1 January 1983 (1983-01-01), pages 1941-1950,

## Description

La présente invention a pour objet la synthèse de polyuréthane par auto-condensation. Elle a également pour objet de nouveaux monomères ainsi que leurs procédés de préparation et leur utilisation pour la synthèse de polyuréthane.

Les polyuréthanes font partie des plus importants matériaux polymériques et présentent de nombreuses propriétés utiles pour diverses applications, comme l'industrie des machines, les revêtements et les peintures, les matériaux d'isolation, les fibres élastiques, les mousses flexibles ou encore les dispositifs médicaux. De plus, la chimie des polyuréthanes permet la synthèse de différents types de matières polymériques comme les mousses (flexibles et rigides), les matières thermoplastiques, les réseaux de polymères interpénétrants (IPN) et les polyuréthanes segmentés, et ce en fonction du polyol, de l'isocyanate et de la méthode de polymérisation mise en oeuvre.

Récemment, on s'est fortement intéressé à la synthèse de polyuréthanes à partir de ressources naturelles comme les huiles végétales et les graisses naturelles en raison de leur forte disponibilité, durabilité, et de leur caractère biodégradable. De plus, ils présentent une meilleure compatibilité écologique par rapport aux produits pétrochimiques. De nombreuses études récentes concernent la synthèse et la caractérisation d'une large gamme de polymères à partir d'huiles végétales (Miyagawa, H.; Misra, M.; Drzala, L. T.; Mohanty, A. K. Polymer, 2005, 46, 445 ; Lu, Y.; Larock, R. C. Biomacromolecules, 2008, 9, 3332 ; Wang, H. J.; Rong, M. Z.; Zhang, M. Q.; Hu, J.; Chen, H. W.; Czigány, T. Biomacromolecules 2008, 9, 615).

Les huiles végétales sont des triglycérides et ont dans leur structure la plupart du temps au moins un acide gras insaturé (Petrovic, Z. Polymer Reviews, 2008, 48, 109). L'utilisation d'enzymes ou de produits chimiques pour modifier la structure de l'acide gras et introduire des groupes fonctionnels est connue depuis longtemps (Leitheiser, R. H.; Peloza, C. C.; Lyon, C., K. J. Cellular Plastics 1969, 5, 346; Ehrich, A.; Smith, M. K.; Patton, T. C. J Am Oil Chem Soc, 1959, 6, 149 ; Sakamoto, W. K.; Kanda, D.H.F.; Andrade, F. D. A.; Das-gupta, D. K. J. Mater. Sci., 2003, 38, 1465; Fan, Q.; Xiao, C. Polymer Composite, 2008, 758 ; Alam, J.; Riaz, U.; Ahmad, S., Polym. Adv. Technol. 2008, 19, 882 ; Zanetti-Ramos, B. G.; Lemos-Senna, E.; Soldi, V.; Borsali, R.; Cloutet, E.; Cramail, H., Polymer, 2006, 47, 8080). De nombreux types d'huiles végétales ont donc été testés pour la synthèse de polyols, comme l'huile de colza, l'huile de tung, l'huile de lin, l'huile de tournesol ou encore l'huile de soja.

Les polyuréthanes sont classiquement préparés par la réaction de polyols avec des isocyanates.

Les isocyanates sont très réactifs et chimiquement toxiques. Il est donc souhaitable de mettre en oeuvre un procédé de préparation de polyuréthanes par une voie sans isocyanates (voie "non-isocyanates").

De nombreux travaux ont été effectués sur la synthèse de nouveaux polyols destinés à la préparation de polyuréthanes mais moins sur la synthèse de polyuréthanes à partir d'huiles végétales sans mettre en oeuvre d'isocyanates.

A ce jour, il existe deux types de procédé par la voie "non-isocyanates". Le premier type de procédé est la méthode d'auto-condensation dans laquelle un monomère de type AB contient des groupes hydroxyles et azotures (N.D.Ghatze et al., J. Polym. Science, vol.21, 1941-1950 (1983); Kumar, A.; Ramakrishnan, S. Chem. Commun., 1993, 1453 ; Kumar, A.; Ramakrishnan, S. J. Polym. Sci.: Part A Polym. Chem., 1996, 34, 839 ; Ranganathan, T.; Ramesh, C.; Kumar, A. Chem. Commun., 2004, 154). L'autre type de procédé est une ouverture de cycle de carbonates cycliques par des amines (Ochiai, B.; Satoh, Y.; Endo, T, J. Polym. Sci. Part A: Polym Chem., 39, 4091, 2001).

La présente invention a donc pour objet de fournir un nouveau procédé de préparation de polyuréthane par une voie sans isocyanates.

La présente invention a pour but de fournir un procédé de préparation de polyuréthane effectué en l'absence d'isocyanates, ledit procédé permettant un contrôle stoechiométrique.

La présente invention a également pour but de fournir de nouveaux monomères de réaction destinés à la préparation de polyuréthanes en l'absence d'isocyanates.

La présente invention concerne des composés de formule (I) suivante : 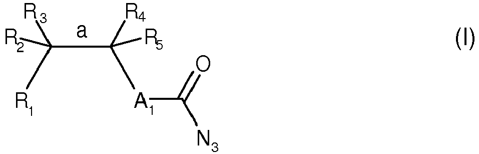 dans laquelle :
- R₁ représente un groupe alkyle, linéaire ou ramifié, comprenant de 1 à 20 atomes de carbone, le cas échéant substitué par un ou plusieurs substituants ORₐ, Rₐ représentant un atome d'hydrogène ou un groupe alkyle comprenant de 1 à 20 atomes de carbone, ledit groupe R₁ pouvant éventuellement contenir une ou plusieurs insaturations ;
- a représente une simple ou une double liaison ;
- R₂ représente un atome d'hydrogène, un groupe ORₐ, Rₐ représentant un atome d'hydrogène ou un groupe alkyle comprenant de 1 à 20 atomes de carbone, ou un groupe alkyle, linéaire ou ramifié, comprenant de 1 à 20 atomes de carbone, substitué par un groupe OH,
   ou R₂ représente un radical de formule -(OCH₂CH₂)ₙ-OH ou -CH₂-(CH₂OCH₂)ₙ-CH₃, n représentant un nombre entier compris de 1 à 100, de préférence de 6 à 50, et préférentiellement égal à 6, 13 ou 45 ;
- R₃ représente, lorsque a est une simple liaison, un atome d'hydrogène ou, lorsque a est une double liaison, R₃ est absent ;
- R₄ représente un atome d'hydrogène ou un groupe OR_{b}, R_{b} représentant un atome d'hydrogène ou un groupe alkyle comprenant de 1 à 20 atomes de carbone, ou un groupe alkyle, linéaire ou ramifié, comprenant de 1 à 20 atomes de carbone, substitué par un groupe OH,
   ou R₄ représente un radical de formule -(OCH₂CH₂)ₙ-OH ou -CH₂-(CH₂OCH₂)ₙ-CH₃, n représentant un nombre entier compris de 1 à 100, de préférence de 6 à 50, et préférentiellement égal à 6, 13 ou 45 ;
- R₅ représente, lorsque a est une simple liaison, un atome d'hydrogène ou, lorsque a est une double liaison, R₅ est absent ; et
- A₁ représente un radical alkylène divalent, linéaire ou ramifié, comprenant de 1 à 20 atomes de carbone, ledit radical alkylène contenant éventuellement une ou plusieurs insaturations.

Dans le cadre de la présente invention, lorsque le radical A₁ comprend une ou plusieurs insaturations, il est possible d'envisager une étape de fonctionnalisation ultérieure du composé de formule (I) susmentionnée. Cette étape permet d'introduire des modifications chimiques sur la ou les insaturations. Ainsi, on peut par exemple envisager une étape d'époxydation puis d'ouverture de cycle, ce qui permet d'introduire des groupements hydroxyles dans la chaîne A₁. De même, lorsque R₁ comprend une ou plusieurs insaturations, il est possible d'envisager une étape de fonctionnalisation ultérieure du composé de formule (I) susmentionnée. Cette étape permet d'introduire des modifications chimiques sur la ou les insaturations. Ainsi, on peut par exemple envisager une étape d'époxydation puis d'ouverture de cycle, ce qui permet d'introduire des groupements hydroxyles dans la chaîne R₁.

Selon la présente invention, les radicaux "alkyle" représentent des radicaux hydrocarbonés saturés, en chaîne droite ou ramifiée, comprenant de 1 à 20 atomes, notamment de 1 à 12 atomes de carbone, de préférence de 1 à 10 atomes de carbone, et encore de préférence de 1 à 5 atomes de carbone (ils peuvent typiquement être représentés par la formule CₙH₂ₙ₊₁, n représentant le nombre d'atomes de carbone). On peut notamment citer, lorsqu'ils sont linéaires, les radicaux méthyle, éthyle, propyle, butyle, pentyle, hexyle, octyle, nonyle et décyle. On peut notamment citer, lorsqu'ils sont ramifiés ou substitués par un ou plusieurs radicaux alkyles, les radicaux isopropyle, tert-butyle, 2-éthylhexyle, 2-méthylbutyle, 2-méthylpentyle, 1-méthylpentyle et 3-méthylheptyle. Le terme "alkyle" désigne également les radicaux cycloalkyles qui sont des radicaux hydrocarbonés mono-, bi- ou tri- cycliques saturés ou partiellement insaturés, non aromatiques, comprenant de 3 à 20 atomes de carbone, et de préférence de 3 à 10 atomes de carbone, tel que notamment le cyclopropyle, cyclopentyle, cyclohexyle ou adamantyle, ainsi que les cycles correspondants contenant une ou plusieurs insaturations.

Les radicaux "alkyle" susmentionnés peuvent être substitués par un ou plusieurs substituants. Parmi ces substituants, on peut citer les groupes suivants : amino, hydroxy, thio, halogène, carboxyle, alkyle, alcoxy, alkylthio, alkylcarbonyle, alkylcarboxyle, alkylamino, aryloxy, arylalkoxy, cyano, trifluorométhyle, alkylsulfonyle, carboxy ou carboxyalkyle.

Les radicaux "alcoxy" selon la présente invention sont des radicaux de formule -O-alkyle, le groupe alkyle étant tel que défini précédemment.

Le terme "alkylthio" désigne un groupe -S-alkyl, le groupe alkyle étant tel que défini ci-dessus.

Le terme "alkylamino" désigne un groupe -NH-alkyl, le groupe alkyle étant tel que défini ci-dessus.

Le terme "alkylcarbonyle" désigne un groupe -CO-alkyl, le groupe alkyle étant tel que défini ci-dessus.

Le terme "alkylcarboxyle" désigne un groupe -COO-alkyl, le groupe alkyle étant tel que défini ci-dessus.

Le terme "alkylsulfonyle" désigne un groupe -SO₂-alkyl, le groupe alkyle étant tel que défini ci-dessus.

Parmi les atomes d'halogène, on cite plus particulièrement les atomes de fluor, de chlore, de brome et d'iode.

Le terme "aryloxy" désigne un groupe -O-aryle, le groupe aryle étant tel que défini ci-dessus.

Le terme "arylalkoxy" désigne un groupe aryl-alkoxy-, les groupes aryles et alkoxy étant tels que définis ci-dessus.

Le terme "carboxyalkyle" désigne un groupe HOOC-alkyl-, le groupe alkyle étant tel que défini ci-dessus. Comme exemple de groupes carboxyalkyles, on peut citer notamment le carboxyméthyle ou le carboxyéthyle.

Lorsqu'un radical alkyle est substitué par un groupe aryle, on parle de radical "arylalkyle" ou "aralkyle". Les radicaux « arylalkyles » ou « aralkyles » sont des radicaux aryl-alkyl-, les groupes aryles et alkyles étant tels que définis ci-dessus. Parmi les radicaux arylalkyles, on peut notamment citer le radical benzyle ou phénéthyle. Ces groupes arylalkyles peuvent être substitués par un ou plusieurs substituants. Parmi ces substituants, on peut citer les groupes suivants : amino, hydroxy, thio, halogène, carboxyle, alkyle, alcoxy, alkylthio, alkylcarbonyle, alkylcarboxyle, alkylamino, aryloxy, arylalkoxy, cyano, trifluorométhyle, alkylsulfonyle, carboxy ou carboxyalkyle.

Selon la présente invention, les radicaux "alkylène" représentent des radicaux (également nommés alkylidènes) dérivés des alcanes dont les deux atomes d'hydrogène terminaux ont été supprimés. Lorsque lesdits radicaux alkylènes sont linéaires, ils peuvent être représentés par la formule -(CH₂)ₙ-.

De préférence, dans la formule (I) susmentionnée, R₁ représente un groupe alkyle, notamment comprenant 8 atomes de carbone.

Dans la formule (I) susmentionnée, R₁ peut également représenter avantageusement un groupe alkyle substitué par un groupe OH, et plus particulièrement un groupe CH₃(CH₂)₅CH(OH)CH₂.

De préférence, dans la formule (I) susmentionnée, R₂ représente un groupe OH.

Dans la formule (I) susmentionnée, R₂ peut également représenter avantageusement H.

De préférence, dans la formule (I) susmentionnée, R₄ représente un groupe alcoxy OR_{b}, R_{b} représentant un groupe alkyle, et plus particulièrement R₄ peut représenter un groupe OMe.

Dans la formule (I) susmentionnée, R₄ peut également représenter avantageusement H.

Selon un mode de réalisation avantageux, la présente invention concerne des composés de formule (I) telle que définie ci-dessus, dans laquelle au moins un des groupes R₁, R₂ et R₄ comprend un groupe OH ou alcoxy.

Selon un autre mode de réalisation avantageux, la présente invention concerne des composés de formule (I) telle que définie ci-dessus, dans laquelle, lorsque a représente une simple liaison, au moins un des groupes R₂ et R₄ comprend un groupe OH ou alcoxy.

La présente invention concerne également les composés préférés de formule (I-1) suivante : 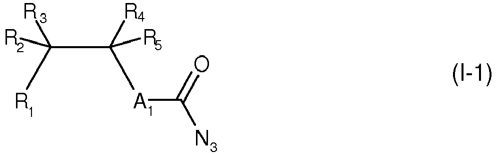 dans laquelle :
- R₁ représente un groupe alkyle, linéaire ou ramifié, comprenant de 1 à 20 atomes de carbone, le cas échéant substitué par un ou plusieurs substituants ORₐ, Rₐ représentant un atome d'hydrogène ou un groupe alkyle comprenant de 1 à 20 atomes de carbone, ledit groupe R₁ pouvant éventuellement contenir une ou plusieurs insaturations ;
- R₂ représente un atome d'hydrogène, un groupe ORₐ, Rₐ représentant un atome d'hydrogène ou un groupe alkyle comprenant de 1 à 20 atomes de carbone, ou un groupe alkyle, linéaire ou ramifié, comprenant de 1 à 20 atomes de carbone, substitué par un groupe OH, ou R₂ représente un radical de formule -(OCH₂CH₂)ₙ-OH ou -CH₂-(CH₂OCH₂)ₙ-CH₃, n représentant un nombre entier compris de 1 à 100, de préférence de 6 à 50, et préférentiellement égal à 6, 13 ou 45 ;
- R₃ représente un atome d'hydrogène ;
- R₄ représente un atome d'hydrogène ou un groupe OR_{b}, R_{b} représentant un atome d'hydrogène ou un groupe alkyle comprenant de 1 à 20 atomes de carbone, ou un groupe alkyle, linéaire ou ramifié, comprenant de 1 à 20 atomes de carbone, substitué par un groupe OH, ou R₄ représente un radical de formule - (OCH₂CH₂)ₙ-OH ou -CH₂-(CH₂OCH₂)ₙ-CH₃, n représentant un nombre entier compris de 1 à 100, de préférence de 6 à 50, et préférentiellement égal à 6, 13 ou 45;
- R₅ représente un atome d'hydrogène ; et
- A₁ représente un radical alkylène divalent, linéaire ou ramifié, comprenant de 1 à 20 atomes de carbone, ledit radical alkylène contenant éventuellement une ou plusieurs insaturations.

Les composés de formule (I-1) correspondent à des composés de formule (I) telle que définie ci-dessus, dans laquelle a représente une liaison simple.

Selon un mode de réalisation avantageux, la présente invention concerne des composés de formule (I-1) telle que définie ci-dessus, dans laquelle au moins un des groupes R₂ et R₄ comprend un groupe OH ou alcoxy.

La présente invention concerne également les composés préférés de formule (I-2) suivante : 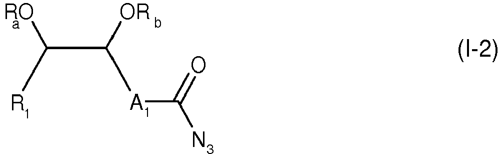 dans laquelle :
- R₁ représente un groupe alkyle, linéaire ou ramifié, comprenant de 6 à 12 atomes de carbone ;
- Rₐ représente un atome d'hydrogène ou un groupe alkyle comprenant de 1 à 12 atomes de carbone ;
- R_{b} représente un atome d'hydrogène ou un groupe alkyle comprenant de 1 à 12 atomes de carbone ; et
- A₁ représente un radical alkylène divalent, linéaire ou ramifié, comprenant de 6 à 12 atomes de carbone.

Les composés de formule (I-2) correspondent à des composés de formule (I) telle que définie ci-dessus, dans laquelle a représente une liaison simple, R₃ est H, R₂ est un groupe ORₐ, R₄ est un groupe OR_{b} et R₅ est H.

Selon un mode de réalisation particulier, la présente invention concerne les composés de formule (I-2) telle que définie ci-dessus, dans laquelle Rₐ est H.

Selon un mode de réalisation particulier, la présente invention concerne les composés de formule (I-2) telle que définie ci-dessus, dans laquelle R_{b} est un groupe méthyle.

Selon un mode de réalisation particulier, la présente invention concerne les composés de formule (I-2) telle que définie ci-dessus, dans laquelle A₁ est un radical alkylène, notamment linéaire, comprenant 7 atomes de carbone.

Selon un mode de réalisation particulier, la présente invention concerne les composés de formule (I-2) telle que définie ci-dessus, dans laquelle R₁ est un groupe alkyle, notamment linéaire, comprenant 8 atomes de carbone.

Ainsi, la présente invention concerne également le composé préféré de formule (I-3) suivante :

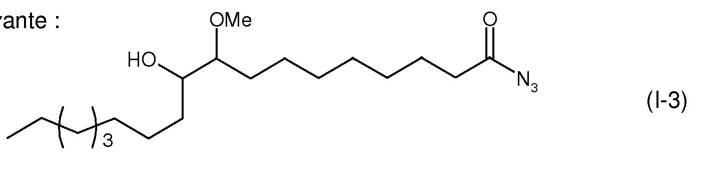

Ce composé est dénommé par la suite HMODAz. Il s'agit de l'azoture de 10-hydroxy-9-méthoxyoctadécanoyle qui est un nouveau monomère de type AB obtenu à partir de l'huile de tournesol.

La présente invention concerne également les composés de formule (I-4) suivante : 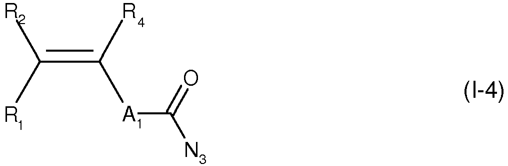 dans laquelle :
- R₁ représente un groupe alkyle, linéaire ou ramifié, comprenant de 1 à 20 atomes de carbone, le cas échéant substitué par un ou plusieurs substituants ORₐ, Rₐ représentant un atome d'hydrogène ou un groupe alkyle comprenant de 1 à 20 atomes de carbone, ledit groupe R₁ pouvant éventuellement contenir une ou plusieurs insaturations ;
- R₂ représente un atome d'hydrogène, un groupe ORₐ, Rₐ représentant un atome d'hydrogène ou un groupe alkyle comprenant de 1 à 20 atomes de carbone, ou un groupe alkyle, linéaire ou ramifié, comprenant de 1 à 20 atomes de carbone, substitué par un groupe OH, ou R₂ représente un radical de formule -(OCH₂CH₂)ₙ-OH ou -CH₂-(CH₂OCH₂)ₙ-CH₃, n représentant un nombre entier compris de 1 à 100, de préférence de 6 à 50, et préférentiellement égal à 6, 13 ou 45 ;
- R₄ représente un atome d'hydrogène ou un groupe OR_{b}, R_{b} représentant un atome d'hydrogène ou un groupe alkyle comprenant de 1 à 20 atomes de carbone, ou un groupe alkyle, linéaire ou ramifié, comprenant de 1 à 20 atomes de carbone, substitué par un groupe OH, ou R₄ représente un radical de formule-(OCH₂CH₂)ₙ-OH ou -CH₂-(CH₂OCH₂)ₙ-CH₃, n représentant un nombre entier compris de 1 à 100, de préférence de 6 à 50, et préférentiellement égal à 6, 13 ou 45;
- A₁ représente un radical alkylène divalent, linéaire ou ramifié, comprenant de 1 à 20 atomes de carbone, ledit radical alkylène contenant éventuellement une ou plusieurs insaturations.

Les composés de formule (I-4) correspondent à des composés de formule (I) telle que définie ci-dessus, dans laquelle a représente une double liaison et R₃ et R₅ sont absents.

De préférence, dans la formule (I-4) susmentionnée, R₂ représente H.

De préférence, dans la formule (I-4) susmentionnée, R₄ représente H.

Ainsi, parmi les composés de formule (I-4) telle que définie ci-dessus, on peut citer les composés de formule (I-5) suivante : 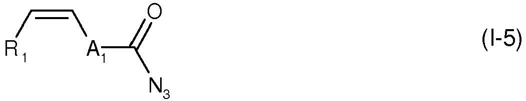 dans laquelle :
- R₁ représente un groupe alkyle, linéaire ou ramifié, comprenant de 6 à 12 atomes de carbone, le cas échéant substitué par un groupe OH,
- A₁ représente un radical alkylène divalent, linéaire ou ramifié, comprenant de 6 à 12 atomes de carbone.

Les composés de formule (I-5) correspondent à des composés de formule (I) telle que définie ci-dessus, dans laquelle a représente une double liaison, R₃ et R₅ sont absents et R₂ et R₄ sont H.

De préférence, dans la formule (I-5) susmentionnée, R₁ est un groupe alkyle substitué par un groupe OH.

Ainsi, la présente invention concerne également des composés de formule (I-6) suivante : 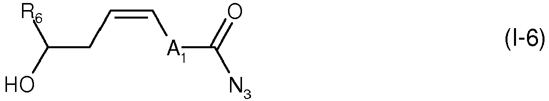 dans laquelle :
- A₁ est tel que défini ci-dessus pour la formule (I-5), et
- R₆ représente un groupe alkyle, linéaire ou ramifié, comprenant de 1 à 12 atomes de carbone.

Parmi les composés préférés de l'invention, on peut citer le composé de formule (I-7) suivante :

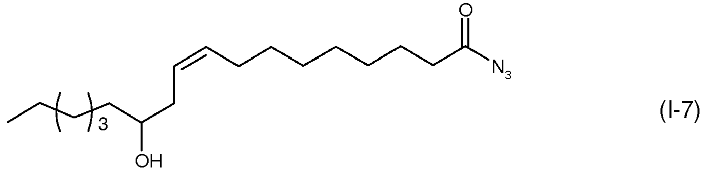

Ce composé est dénommé par la suite HODEAz. Il s'agit de l'azoture de 12-hydroxy-octadec-9-écanoyle qui est un nouveau monomère de type AB obtenu à partir de l'huile de ricin.

La présente invention concerne également un procédé de préparation d'un composé de formule (I) telle que définie ci-dessus, comprenant une étape de réaction d'un composé de formule (II) suivante : 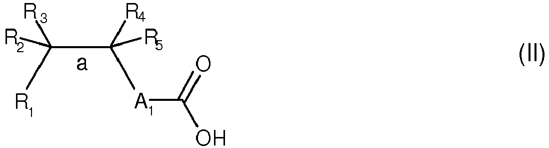 dans laquelle R₁, R₂, R₃, R₄, R₅, A₁ et a sont tels que définis ci-dessus pour la formule (I),
avec du chloroformate d'éthyle en présence de triéthylamine, puis avec de l'azoture de sodium.

De préférence, l'étape de réaction du composé de formule (II) susmentionnée est effectuée en deux étapes : la première étape consiste à faire réagir le composé (II) avec le chloroformate d'éthyle en présence de triéthylamine dans un mélange THF/eau pendant 2 heures à 0°C et la deuxième étape consiste ensuite à faire réagir le composé intermédiaire obtenu à l'issue de la première étape avec de l'azoture de sodium dans de l'eau pendant 4 heures à 0°C.

La présente invention concerne également l'utilisation d'un composé de formule (I) telle que définie ci-dessus, pour la préparation de polyuréthane.

Plus particulièrement, la présente invention concerne l'utilisation d'un composé de formule (I-2) ou (I-3), pour la préparation de polyuréthane répondant à la formule (III-1) suivante : 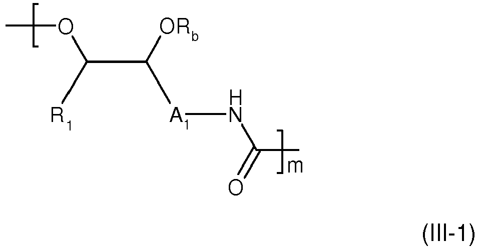 A₁, R₁ et R_{b} étant tels que définis ci-dessus pour la formule (I-2), et
m représentant un nombre entier compris de 2 à 50 000, et de préférence de 2 à 10 000, notamment de 2 à 5 000, et encore de préférence de 2 à 50.

Plus particulièrement, la présente invention concerne l'utilisation d'un composé de formule (I-3) telle que définie ci-dessus, pour la préparation de polyuréthane répondant à la formule (III-1-1) suivante : 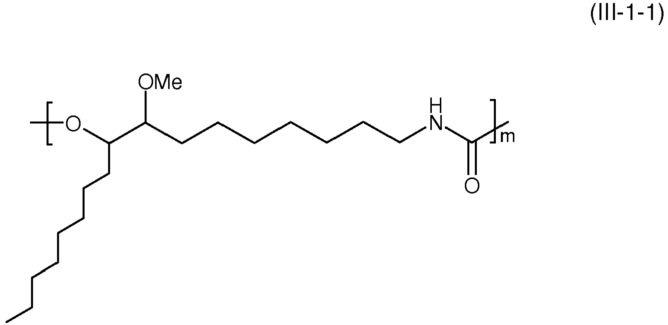 m représentant un nombre entier compris de 2 à 50 000, et de préférence de 2 à 10 000, notamment de 2 à 5 000, et encore de préférence de 2 à 50.

La présente invention concerne également l'utilisation d'un composé de formule (I), et plus particulièrement d'un composé de formule (I-4), (I-5), (I-6) ou (I-7), pour la préparation de polyuréthane répondant à la formule (III-2) suivante : 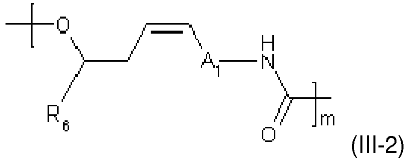 R₆ et A₁ étant tels que définis ci-dessus pour la formule (I-6), et
m représentant un nombre entier compris de 2 à 50 000, et de préférence de 2 à 10 000, notamment de 2 à 5 000, et encore de préférence de 2 à 50.

Plus particulièrement, la présente invention concerne l'utilisation d'un composé de formule (I-7) telle que définie ci-dessus, pour la préparation de polyuréthane répondant à la formule (III-2-1) suivante : 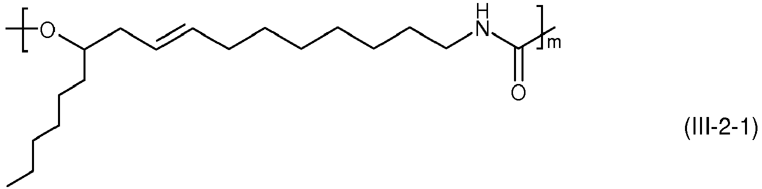
m représentant un nombre entier compris de 2 à 50 000, et de préférence de 2 à 10 000, notamment de 2 à 5 000, et encore de préférence de 2 à 50.

La présente invention concerne également un procédé de préparation de polyuréthane, notamment répondant à l'une quelconque des formules (III-1), (III-1-1), (III-2) ou (III-2-1), comprenant une étape d'auto-condensation d'un composé de formule (I) telle que définie ci-dessus à une température comprise de 50°C à 100°C, de préférence à 80°C, pendant une durée de 1 heure à 48 heures, de préférence pendant 24 heures.

La présente invention concerne également un procédé de préparation de polyuréthane, notamment répondant à l'une quelconque des formules (III-2) ou (III-2-1), comprenant les étapes suivantes :
- une étape de réaction d'un composé de formule (I) telle que définie ci-dessus avec un alcool R₇OH, R₇ représentant un groupe alkyle, linéaire ou ramifié, comprenant de 1 à 4 atomes de carbone, R₇ étant de préférence un groupe méthyle, pour obtenir un composé de formule (IV) suivante : 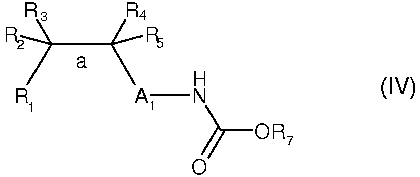 dans laquelle R₁, R₂, R₃, R₄, R₅, A₁ et a sont tels que définis ci-dessus dans la formule (I), et
- une étape de polycondensation du composé de formule (IV) en présence de tétrabutoxyde de titane à une température comprise de 80°C à 180°C, de préférence à 130°C, pendant une durée de 2 heures à48 heures, de préférence pendant 6 heures.

De préférence, la première étape de ce procédé est effectuée à reflux pendant 4 heures.

### DESCRIPTION DES FIGURES

La **Figure 1** représente un spectre FTIR (Spectroscopie Infrarouge à Transformée de Fourier) d'échantillons de polymérisation du HMODAz (5) après différents temps de polymérisation.
La **Figure 2** représente le spectre RMN du proton (¹H-RMN) d'échantillons de polymérisation du HMODAz (5) à différents intervalles de temps.
La **Figure 3** représente les analyses effectuées par SEC (chromatographie d'exclusion stérique) d'échantillons de polymérisation du HMODAz (composé 5) en fonction du temps.
La **Figure 4** représente les thermogrammes DSC ("Differential Scanning Calorimetry" - Calorimétrie à balayage différentiel) des polyuréthanes (6)(composé de formule (III-1-1)) et (9)(composé de formule (III-2-1)).
La **Figure 5** représente le spectre RMN du proton (¹H-RMN) du polyuréthane dérivé de l'acide ricinoléique (11).

### EXEMPLES

### Exemple 1 : Synthèse du HMODAz - composé de formule (I-3)

Le composé HMODAz (composé 5 ci-après) a été préparé selon le schéma réactionnel décrit ci-après : 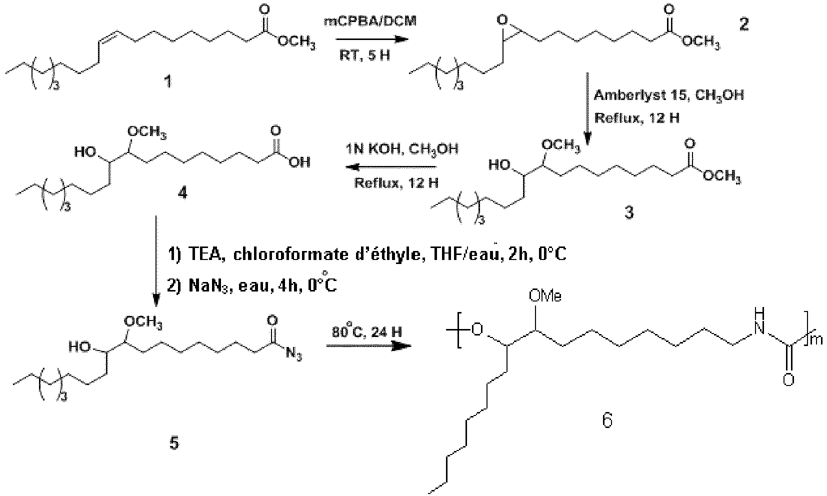 Le monomère (5) est obtenu à partir d'une source naturelle : l'huile de tournesol qui est transestérifié avec du méthanol en présence de MgO comme catalyseur.

Ce monomère HMODAz est synthétisé selon un procédé en cinq principales étapes.

### Synthèse du 8-(3-octyloxiran-2-yl)octanoate de méthyle (2)

L'oléate de méthyle (1) (5,0 g; 0,017 mol)(89%)(ITERG) et l'acide méta-chloroperbenzoïque (mCPBA)(4,3 g ; 0,025 mol)(Aldrich) ont été dissous dans le dichlorométhane (J. T. Baker)(100 mL) et le mélange réactionnel a été mélangé sous agitation à température ambiante pendant 5 heures. Le mélange réactionnel a ensuite été filtré et la solution récupérée a été lavée avec du bicarbonate de sodium aqueux saturé (3 x 50 mL)(Aldrich) puis avec de l'eau (3 x 50 mL). La couche organique a été séparée et séchée avec du sulfate de sodium anhydre. Enfin, le solvant a été éliminé par évaporation sous rotation pour obtenir le composé intermédiaire 2.
Rendement : 4,8 g (91 %)
IR : 1740 cm⁻¹ (COOCH₃), 841 cm⁻¹ (époxy).
¹H-RMN (400 MHz, CDCl₃) : 0,87 (3H, t, C*H*₃) ; 1,20-1,70 (protons méthylène) ; 2,29 (2H, t, C*H*₂-COOCH₃) ; 2,99 (2H, protons du cycle époxy) ; 3,66 (3H, s, COOC*H*₃).

### Synthèse du 10-hydroxy-9-méthoxyoctadécanoate de méthyle (3)

Le 8-(3-octyloxiran-2-yl)octanoate (2) de méthyle (4,8 g ; 0,015 mol), de l'Amberlyst 15 (0,05 g) et du méthanol en excès (100 mL) ont été mis à reflux pendant 12 heures. Le mélange réactionnel a été filtré et le méthanol a été éliminé en utilisant un évaporateur rotatif. Le mélange réactionnel a été dissous dans du dichlorométhane (100 mL) et lavé avec de l'eau (3 x 50 mL). La solution de dichlorométhane a été séparée et évaporée pour obtenir le composé intermédiaire 3.
Rendement : 4,7 g (89%)
IR : 3476 cm⁻¹ (OH), 1740 cm⁻¹ (COOCH₃),
¹H-RMN (400 MHz, CDCl₃) : 0,88 (3H, t, C*H*₃) ; 1,20-1,70 (protons méthylène) ; 2,29 (2H, t, C*H*₂-COOH) ; 2,99 (1 H, q, C*H*-OCH₃) ; 3,41 (3H, s, OC*H*₃) ; 3,49 (1 H, m, C*H*-OH) ; 3,66 (3H, s, COOC*H*₃).

### Synthèse de l'acide 10-hydroxy-9-méthoxyoctadécanoïque (4)

Le 10-hydroxy-9-méthoxyoctadécanoate de méthyle (3) (4,0 g ; 0,012 mol) a été dissous dans une solution d'hydroxyde de potassium méthanolique 1 N (100 mL)(Aldrich) et chauffé à reflux pendant 12 h. Le méthanol a été éliminé du mélange réactionnel et le produit brut a été dissous dans 100 mL d'eau. La solution aqueuse a été neutralisée avec de l'acide chlorhydrique (Aldrich) et le produit a été extrait avec 3 x 50 mL de dichlorométhane (J.T.Baker). La solution de dichlorométhane a été lavée avec 3 x 50 mL d'eau et séchée sur du sulfate de sodium anhydre (J.T.Baker). La solution de dichlorométhane a été filtrée et le solvant a été éliminé pour obtenir le composé intermédiaire 4.
Rendement : 3,50 g (91%)
IR : 3435 cm⁻¹ (OH), 1709 cm⁻¹ (COOH).
¹H-RMN (400 MHz, CDCl₃) : 0,86 (3H, t, C*H*₃) ; 1,20-1.70 (protons méthylène) ; 2,34 (2H, t, C*H*₂-COOH) ; 2,98 (1 H, q, C*H*-OCH₃) ; 3,39 (3H, s, OC*H*₃) ; 3,47 (1 H, m, C*H*-OH).
¹³C-RMN (400 MHz, CDCl₃) : 179,32 ppm (COOH) ; 84,41 ppm (CH-OMe) ; 72,20 ppm (CH-OH) ; 58,31 ppm (OCH₃) ; 22-35 ppm (protons de la chaîne alkyle) et 13,90 ppm (CH₃).

### Synthèse du monomère de type AB : azoture de 10-hydroxy-9-méthoxyoctadécanoyle (HMODAz)(5)

Dans un ballon à fond rond de 100 mL équipé d'un agitateur magnétique et d'une ampoule de coulée, on a introduit l'acide 10-hydroxy-9-méthoxyoctadécanoïque (4) (2,0 g ; 0,006 mol), la triéthylamine (Aldrich)(1,8 g ; 0,018 mol) et le mélange THF:eau (7:3 v/v, 30 mL). Le mélange réactionnel a été refroidi à 0°C et on a ajouté goutte à goutte pendait 10 minutes du chloroéthyl formate (Fluka)(1,96 g ; 0,018 mol). Le mélange réactionnel a été agité pendant 2 heures puis l'azoture de sodium (Aldrich)(1,2 g ; 0,018 mol) dans de l'eau (7 mL) a été ajouté goutte à goutte pendant 10 minutes et agité à 0°C pendant 4 heures. Le THF a été éliminé sur un évaporateur rotatif et le produit brut récupéré a été dissous dans du dichlorométhane (100 mL). La solution de dichlorométhane a été lavée avec de l'eau (2 x 50 mL), séchée sur du sulfate de sodium anhydre (J.T.Baker), filtrée et le solvant a été éliminé pour obtenir le composé HMODAz (5).
Rendement : 2,0 g (92 %)
IR : 3465 cm⁻¹ (OH), 2138 cm⁻¹ (N₃), 1720 cm⁻¹ (CO).
¹H-RMN (400 MHz, CDCl₃) : 0,87 (3H, t, C*H*₃) ; 1,20-1,70 (protons méthylène) ; 2,32 (2H, t, C*H*₂-CON₃) ; 2,98 (1 H, q, C*H*-OCH₃) ; 3,39 (3H, s, OC*H*₃) ; 3,47 (1 H, m, C*H*-OH).
¹³C-NMR (400 MHz, CDCl₃) : 180,09 (CON₃) ; 84,11 (CH-OMe) ; 72,23 (CH-OH) ; 57,73 (OCH₃) ; 21-37 (protons de la chaîne alkyle) et 13,54 (CH₃).

Le monomère HMODAz (5) est un monomère auto-condensable avec un groupe hydroxyle secondaire et un groupe azoture d'acyle comme précurseur de la fonction isocyanate.

### Polymérisation par auto-condensation de type AB du HMODAz (5)

Dans un ballon à fond rond bicol de 50 ml équipé d'un agitateur magnétique et d'une entrée d'azote, on a introduit de l'azoture de 10-hydroxy-9-méthoxyoctadécanoyle (5) (2,0 g ; 0,005 mol) et on a maintenu l'ensemble dans un bain d'huile à différentes températures (50°C, 60°C 80°C et 110°C) pendant différentes durées.
Rendement : 1,80 g (90%)
IR : 3 338 cm⁻¹ (NH), 1695 cm⁻¹ (CO), 1 526 cm⁻¹ (déformation NH), 1 230 cm⁻¹ (C-N).
¹H-RMN (400 MHz, CDCl₃) : 0,87 (C*H*₃) ; 1,20-1,70 (protons méthylène) ; 3,18 (-C*H*₂-NHCOO) ; 3,39 (OC*H*₃) ; 4,88 (N*H*).

### Exemple 2 : Synthèse du HODEAz - composé de formule (I-7)

Le composé HODEAz (composé 8 ci-après) a été préparé selon le schéma réactionnel décrit ci-après :

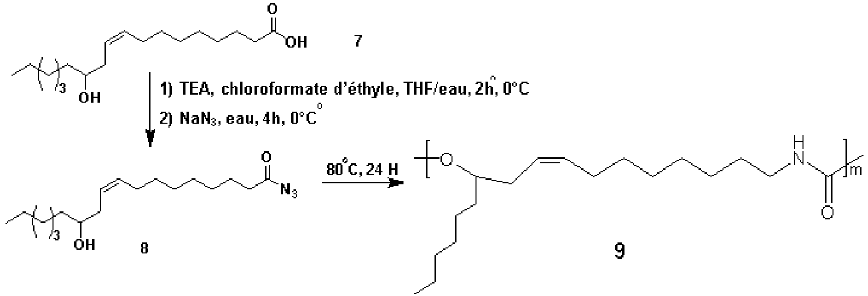

### Synthèse du monomère de type AB : azoture de 12-hydroxy-octadéc-9-énoyle (HODEAz) (8)

Une procédure similaire utilisée pour la synthèse du HMODAz (5) (cf. exemple 1) a été appliquée à la synthèse du monomère (8) HODEAz de type AB à partir d'acide ricinoléique (80%)(TCI chemicals).

L'acide ricinoléique est un acide gras en C18 comprenant un groupe OH avec une double liaison en C9 en configuration cis.
Rendement : 90 %
IR : 3400 cm⁻¹ (OH), 3008 (=C-H), 2133 cm⁻¹ (N₃), 1720 cm⁻¹ (CO).
¹H-RMN (400 MHz, CDCl₃) : 0,87 (3H, t, C*H*₃) ; 1,20-1,70 (protons méthylène) ; 2,31 (2H, t, C*H*₂-CON₃) ; 3,58 (1 H, m, C*H*-OH) ; 5,3-5,6 (C*H*=C*H*).
¹³C-RMN (400 MHz, CDCl₃) : 180,53 (CON₃) ; 132,96 et 125,31 (CH=CH) ; 71,38 (CH-OH); 42,88 (CH₂CON₃); 36,78 et 35,25 (CH₂-CH(OH)-CH₂); 21-37 (protons de la chaîne alkyle) et 13,54 (CH₃).

### Polymérisation par auto-condensation de type AB du HODEAz (8)

Pour la polymérisation conduisant aux polymères de formule (9), on suit la procédure telle que celle indiquée ci-dessus dans l'exemple 1 pour le polymère de formule (6).

### Exemple 3 : Auto-condensation de HMODAz, caractérisation et contrôle de l'avancement de la réaction

La synthèse de polyuréthanes selon la présente invention est basée sur l'auto-condensation de l'azoture de sodium et des groupes hydroxyles. Cette technique présente les avantages suivants : i) la réaction s'effectue doucement par simple chauffage du monomère selon un procédé 'one pot' et ii) aucune optimisation de ratios molaires n'est nécessaire.

L'auto-condensation du monomère (5) HMODAz a été effectuée à différentes températures comme 50 °C, 60 °C, 80 °C et 110 °C. Le tableau 1 ci-après indique les conditions détaillées de la réaction et les résultats de la polymérisation.

**Tableau 1 - Conditions expérimentales et résultats pour la polymérisation de HMODAz (composé 5)**

| Essai | Température (°C) | Durée (h) | SEC^{b} | | |
|---|---|---|---|---|---|
| | | | Mₙ | M_{w} | M_{w}/Mₙ |
| 1 | 50 | 20 | 3 210 | 4 880 | 1,52 |
| 2^{c} | 50 | 5 | 2 720 | 4 280 | 1,57 |
| 3 (i) | 60 | 8 | 1 040 | 1 620 | 1,55 |
| 3 (ii) | 60 | 23 | 2475 | 3 780 | 1,52 |
| 4 (i) | 80 | 1 | 740 | 1 740 | 2,33 |
| 4 (ii) | 80 | 2 | 1 640 | 3 240 | 1,97 |
| 4 (qui) | 80 | 3 | 2 510 | 4 820 | 1,92 |
| 4 (iv) | 80 | 5 | 2 610 | 3 880 | 1,48 |
| 4 (v) | 80 | 7 | 3 510 | 6 030 | 1,71 |
| 4 (vi) | 80 | 9 | 3 660 | 6 340 | 1,73 |
| 4 (vii) | 80 | 23 | 6 390 | 10 450 | 1,63 |
| 5^{c} | 80 | 6 | 8 890 | 18 740 | 2,10 |
| 6 | 110 | 4 | 3 500 | 6 310 | 1,80 |

| | | | | | |
|---|---|---|---|---|---|
| b- basée sur une calibration PS ; c- réaction en présence de DBTDL (0,1% en poids par rapport à HMODAz) comme catalyseur | | | | | |

On a ainsi constaté que toutes les températures utilisées pour les polymérisations sont efficaces. Il existe un effet crucial de la température sur la décomposition de l'azoture d'acyle ainsi que sur la vitesse de polymérisation. Ainsi, en comparant l'essai n°1 (effectué à 50°C) avec l'essai 4(vii)(effectué à 80°C), on constate qu'on obtient des masses moléculaires supérieures en travaillant à 80°C.

Pour une analyse plus complète de la progression de la réaction, on a effectué différents essais de polymérisation à 80°C

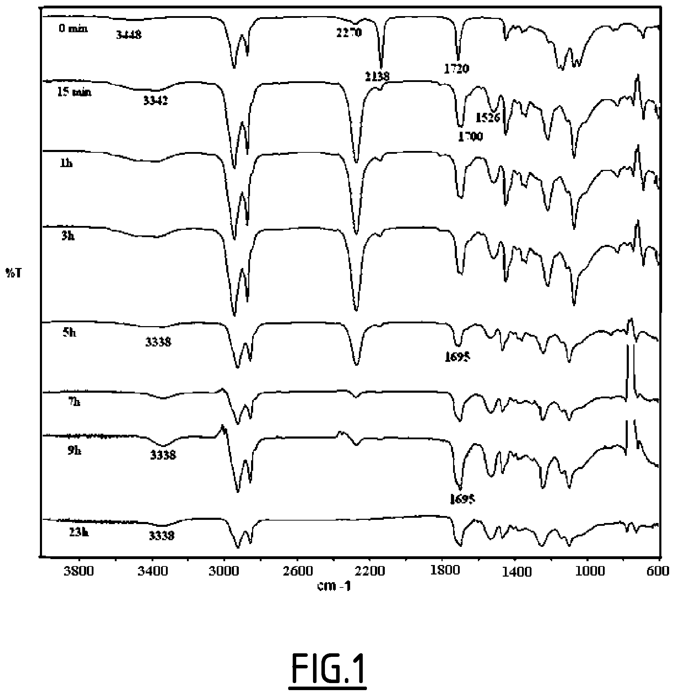

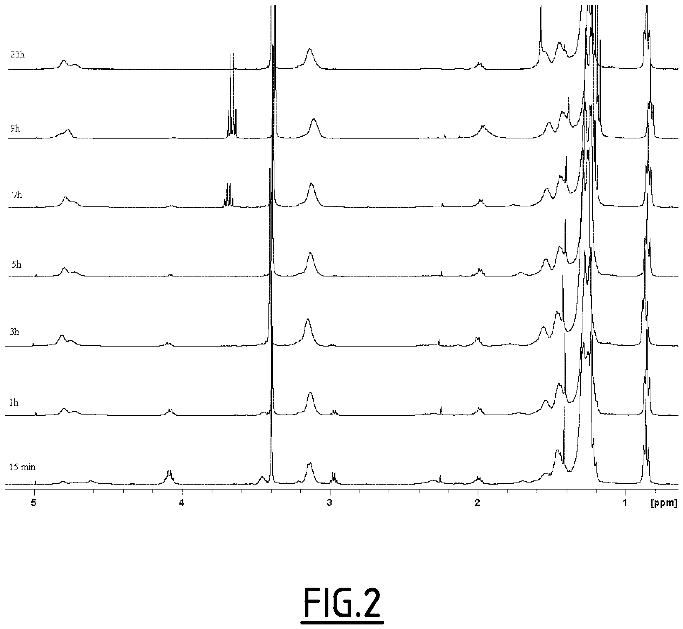

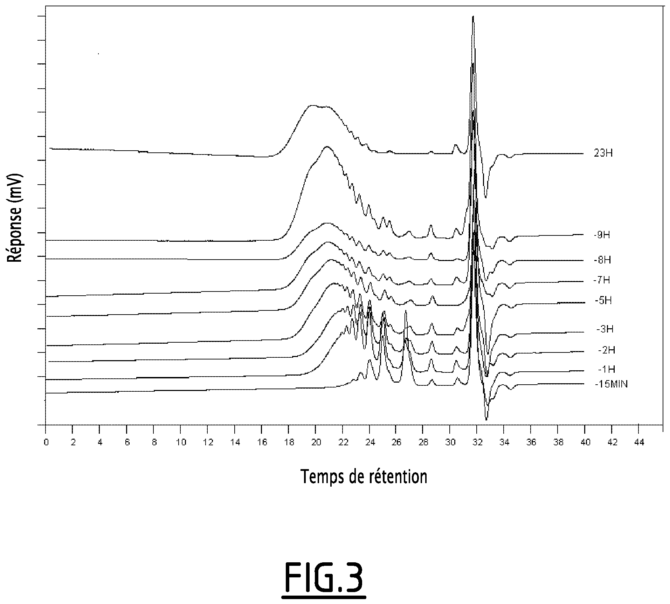

Les Figures 1 et 2 représentent respectivement les spectres FTIR et ¹H-RMN de la progression de la polymérisation et la Figure 3 représente le spectre SEC du contrôle de la réaction.

La bande à 2 138 cm⁻¹ due à la présence du groupe azoture d'acyle (CON₃) est remplacée par une bande à 2 270 cm⁻¹ correspondant aux fonctions isocyanates lors du chauffage à 80°C. La bande d'absorption à 3341 cm⁻¹ est observée en raison de la liaison uréthane N-H. Les échantillons prélevés après 15 minutes d'avancement de la réaction montrent une transformation quasi-complète du groupe acyl-azido en groupe isocyanate d'après le spectre IR (Figure 2). Les échantillons après analyse FTIR ont été mis à réagir avec de l'éthanol et analysés par spectroscopie ¹H-RMN. On a alors observé un pic à 4,06 ppm (Hg) en raison de la présence des protons du groupe méthylène lié au groupe uréthane (Figure 2).

Les spectres FTIR des échantillons montrent que lorsque le temps de polymérisation augmente, l'intensité de la bande isocyanate (2 270 cm⁻¹) diminue en conséquence tandis que l'intensité de la bande N-H (3 338 cm⁻¹) augmente en raison de la formation de polyuréthane. La bande du groupe carbonyle de l'azoture d'acyle à 1 720 cm⁻¹ se déplace à des fréquences inférieures (1 695 cm⁻¹) en raison de la formation de la liaison uréthane.

Des conclusions similaires sont obtenues des spectres ¹H-RMN. L'intensité du pic à 4,06 ppm concernant les protons méthylène attachés au groupe carbamate (NH-COO-C*H₂*CH*₃*) comme groupe terminal diminue au cours du temps (15 min, 1 h, 3h, etc.) en raison de l'augmentation de la masse moléculaire du polyuréthane (Figure 2). On observe également une augmentation des intensités des pics à 4,80 ppm en raison des protons N*H* des groupes uréthanes et un pic à 3,16 ppm en raison des protons du radical méthylène lié au groupe uréthane du polyuréthane (-C*H*₂-NH-COO-).

Les résultats de chromatographie SEC montrent qu'un échantillon obtenu après 15 min présente la formation de traces de di-, tri- et tétramère ainsi que plus de fraction de monomère isocyanate (Figure 3). Alors que le temps de polymérisation augmente, les intensités des pics SEC correspondant aux oligomères diminuent et les pics SEC se déplacent vers des masses moléculaires plus élevées, ce qui illustre le mécanisme de polycondensation.

### Exemple 4 : Auto-condensation de HODEAz, caractérisation et contrôle de l'avancement de la réaction

L'auto-condensation du monomère (8) HODEAz a été effectuée à différentes températures comme 60 °C et 80 ° C. Le tableau 2 ci-après indique les conditions détaillées de la réaction et les résultats de la polymérisation.

**Tableau 2 - Conditions expérimentales et résultats pour la polymérisation de HODEAz (composé 8)**

| Essai | Température (°C) | Durée (h) | SEC^{b} | | |
|---|---|---|---|---|---|
| | | | Mₙ | M_{w} | M_{w}/Mₙ |
| 7 | 60 | 24 | 5 300 | 7 770 | 1,47 |
| 8 | 80 | 10 | 6 210 | 9 330 | 1,50 |
| 9 | 80 | 24 | 6 880 | 10 030 | 1,46 |

| | | | | | |
|---|---|---|---|---|---|
| b- basée sur une calibration PS | | | | | |

On constate d'après ce tableau que la masse moléculaire du polyuréthane synthétisé à 60°C est inférieure à celle du polyuréthane obtenu par auto-condensation à 80°C.

### Exemple 5 : Analyse par calorimétrie à balayage différentiel

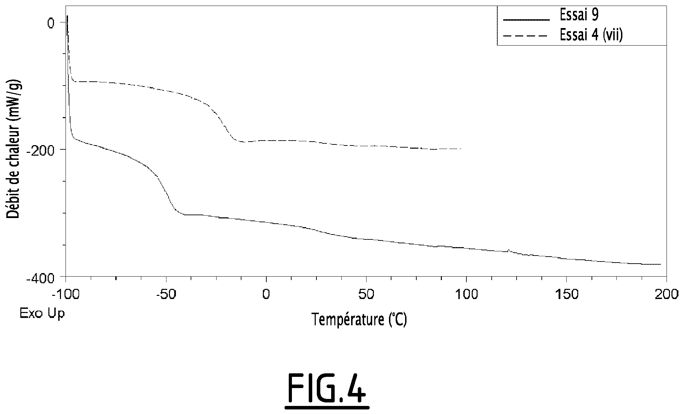

La Figure 4 représente des thermogrammes DSC pour les polyuréthanes dérivés de HMODAz (5) et de HODEAz (8) par auto-condensation à une vitesse de chauffage de 10°C/min. La température de transition vitreuse du polyuréthane synthétisé à partir de HMODAz est -20°C et la température de transition vitreuse du polyuréthane synthétisé à partir de HODEAz est -48°C. Il est donc intéressant de constater que ces polyuréthanes ont des températures de transition vitreuse faibles (en dessous de la température ambiante). De tels polyuréthanes peuvent donc être couplés avec d'autres polymères rigides à blocs pour préparer des élastomères thermoplastiques.

### Exemple 6 : Synthèse de polyuréthane à partir d'acide ricinoléique

Le monomère de type AB (composé 10 ci-après) a été préparé selon le schéma réactionnel décrit ci-après :

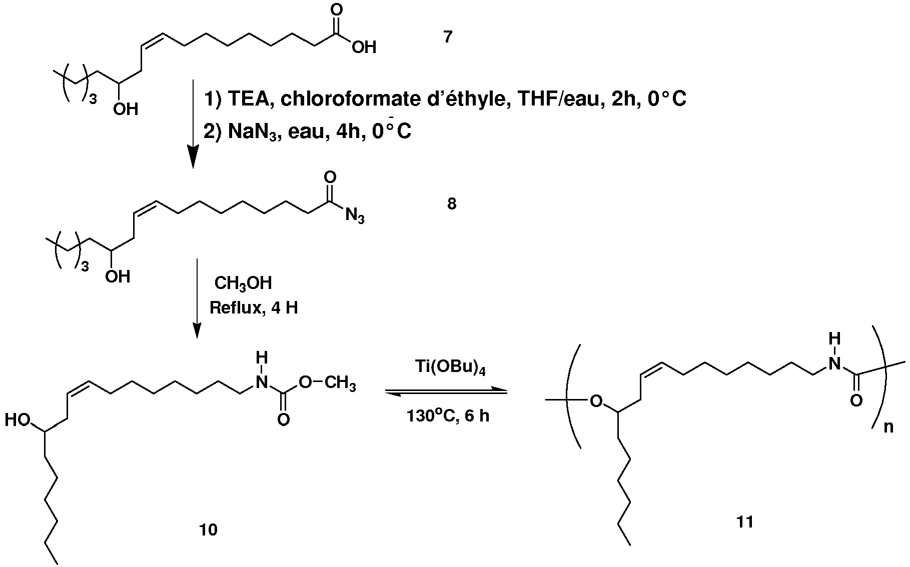

### Synthèse du méthyl-11-hydroxyheptdec-8-énylcarbamate (10), monomère de type AB pour la transuréthanisation

Dans un ballon à fond rond de 100 mL équipé d'un agitateur magnétique et d'un condenseur à reflux, on a introduit de l'azoture de 12-hydroxy-octadec-9-énoyle (8)(2,0 g ; 0,006 mol) et du méthanol sec (50 mL). Le mélange réactionnel a été mis à reflux pendant 4 h puis l'excès de méthanol a été éliminé via un évaporateur rotatif. Le produit carbamate brut a été dissous dans du dichlorométhane (100 mL), lavé avec de l'eau (2 x 50 mL), séché sur du sulfate de sodium anhydre, filtré et le solvant a été éliminé pour obtenir le carbamate (10).
Rendement : 1,85 g (92%)
IR- 3600-3400 cm⁻¹ (OH), 3334 (NH), 3008 (=C-H), 1703 cm⁻¹ (CO).
¹H-RMN (400 MHz, CDCl₃)- 0,87 (3H, t, C*H*₃) ; 1,20-1,70 (protons méthylène) ; 3,14 (2H, C*H*₂-NHCOO-) ; 3,59 (1H, m, C*H*-OH) ; 4,63 (1H, N*H*-COO); 5,3-5,6 (C*H*=CH).

### Polycondensation (via transuréthanisation) pour obtenir le composé (11)

Dans un ballon bicol à fond rond de 50 ml équipé d'un agitateur magnétique, d'un adaptateur pour le vide et d'une entrée d'azote, on a introduit du méthyl-11-hydroxyheptdec-8-énylcarbamate (1,0 g ; 0,003 mol) et du tétrabutoxyde de titane (0,035 g ; 1 x 10⁻⁴ mol). Le mélange réactionnel a été purgé avec de l'azote et mis ensuite sous vide deux fois. Le ballon réactionnel a été maintenu dans un bain d'huile à 130°C pendant 4 heures sous purge d'azote puis sous vide à 130°C pendant 2 heures.
Rendement : 0,80 g (80%)
IR- 3338 cm⁻¹ (NH), 1695 cm⁻¹ (CO), 1526 cm⁻¹ (déformation NH), 1230 cm⁻¹ (C-N).
¹H-RMN (400 MHz, CDCl₃) : 0,86 (C*H*₃) ; 1,20-1,70 (protons méthylène) ; 3,13 (-C*H*₂-NHCOO) ; 3,63 (OC*H*₃) ; 4,86 (N*H*) ; 5,3-5,6 (C*H*=CH).

La transuréthanisation est une voie sans isocyanates et sans solvant utilisée pour la synthèse de polyuréthanes. Elle consiste en une condensation entre des bisuréthanes (A-A) et des diols (B-B) en présence d'un catalyseur. Toutefois, une autre voie consiste à mettre en oeuvre le procédé de l'invention, à savoir une condensation d'un monomère de type AB en présence d'un catalyseur, ledit monomère étant le composé (10): méthyl-11-hydroxyheptdec-8-énylcarbamate (10).

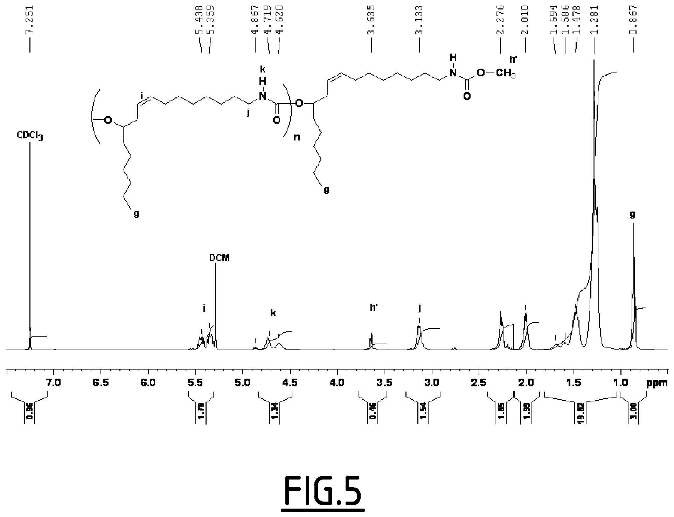

La polycondensation par transuréthanisation a été effectuée à 130°C en présence de tétrabutoxyde de titane en tant que catalyseur en deux étapes. Dans la première étape, la réaction a été effectuée à 130°Cpar agitation sous débit d'azote pendant 4 h pour obtenir des oligomères. La deuxième étape a été effectuée à 130°C sous vide pendant 2 h pour une condensation supplémentaire des oligomères. Le polymère obtenu a été caractérisé par IR, RMN et CPG. Le tableau 3 ci-après indique les conditions de réaction et les résultats de la réaction de transuréthanisation. La réaction de transuréthanisation n'a pas permis d'augmenter la masse moléculaire en l'absence de catalyseur. Le spectre ¹H-RMN du polyuréthane (11) confirme la structure du polyuréthane (Figure 5). La réaction de transuréthanisation a été confirmée sur la base d'une diminution de l'intensité des protons de OCH₃ à 3,65 ppm en comparaison avec une intensité de signal due aux protons -CH₂NHCOO- à 3,13 ppm. A la température de réaction, la présence de double liaison dans le polymère suggéré qu'il n'y a pas de réactions secondaires dues à la double liaison.

**Tableau 3 - Conditions expérimentales et résultats pour la transuréthanisation (composé 11)**

| Essai | Catalyseur | Température (°C) | Durée (h) | SEC^{b} | | |
|---|---|---|---|---|---|---|
| | | | | Mₙ | M_{w} | M_{w}/Mₙ |
| 1 | (Ti(OBu)₄ | 130 | 6 | 6 950 | 9 850 | 1,41 |
| 2^{c} | aucun | 130 | 6 | - | - | - |

| | | | | | | |
|---|---|---|---|---|---|---|
| b- basée sur une calibration PS ; c- aucun polymère | | | | | | |

## Revendications

1. Composé de formule (I) suivante : 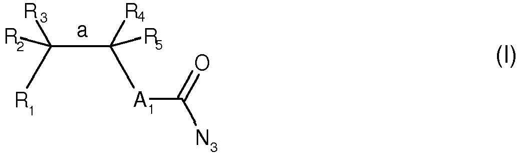 dans laquelle :
- R₁ représente un groupe alkyle, linéaire ou ramifié, comprenant de 1 à 20 atomes de carbone, le cas échéant substitué par un ou plusieurs substituants ORₐ, Rₐ représentant un atome d'hydrogène ou un groupe alkyle comprenant de 1 à 20 atomes de carbone, ledit groupe R₁ pouvant éventuellement contenir une ou plusieurs insaturations, ou R1 représente un radical cycloalkyle mono-, bi- ou tri-cyclique saturé ou partiellement insaturé, non aromatique, comprenant de 3 à 20 atomes de carbone ;
- a représente une simple ou une double liaison ;
- R₂ représente un atome d'hydrogène, un groupe ORₐ, Rₐ représentant un atome d'hydrogène ou un groupe alkyle comprenant de 1 à 20 atomes de carbone, ou un groupe alkyle, linéaire ou ramifié, comprenant de 1 à 20 atomes de carbone, substitué par un groupe OH,
ou R₂ représente un radical de formule -(OCH₂CH₂)ₙ-OH ou -CH₂-(CH₂0CH₂)ₙ-CH₃,
n représentant un nombre entier compris de 1 à 100, de préférence de 6 à 50, et préférentiellement égal à 6, 13 ou 45 ;
- R₃ représente, lorsque a est une simple liaison, un atome d'hydrogène ou, lorsque a est une double liaison, R₃ est absent ;
- R₄ représente un atome d'hydrogène ou un groupe OR_{b}, R_{b} représentant un atome d'hydrogène ou un groupe alkyle comprenant de 1 à 20 atomes de carbone, ou un groupe alkyle, linéaire ou ramifié, comprenant de 1 à 20 atomes de carbone, substitué par un groupe OH,
ou R₄ représente un radical de formule -(OCH₂CH₂)ₙ-OH ou -CH₂-(CH₂OCH₂)ₙ-CH₃,
n représentant un nombre entier compris de 1 à 100, de préférence de 6 à 50, et préférentiellement égal à 6, 13 ou 45 ;
- R₅ représente, lorsque a est une simple liaison, un atome d'hydrogène ou, lorsque a est une double liaison, R₅ est absent ; et
- A₁ représente un radical alkylène divalent, linéaire ou ramifié, comprenant de 1 à 20 atomes de carbone, ledit radical alkylène contenant éventuellement une ou plusieurs insaturations,
dans laquelle au moins un des groupes R₁, R₂ et R₄ comprend un groupe OH ou alcoxy.

2. Composé selon la revendication 1, de formule (I) dans laquelle, lorsque a représente une simple liaison, au moins un des groupes R₂ et R₄ comprend un groupe OH ou alcoxy.

3. Composé selon l'une quelconque des revendications 1 à 2, de formule (I-2) suivante : 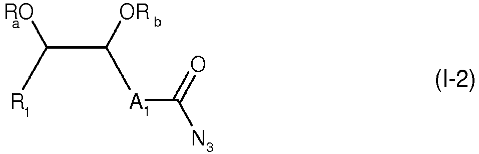 dans laquelle :
- R₁ représente un groupe alkyle, linéaire ou ramifié, comprenant de 6 à 12 atomes de carbone,
- Rₐ représente un atome d'hydrogène ou un groupe alkyle comprenant de 1 à 12 atomes de carbone,
- R_{b} représente un atome d'hydrogène ou un groupe alkyle comprenant de 1 à 12 atomes de carbone, et
- A₁ représente un radical alkylène divalent, linéaire ou ramifié, comprenant de 6 à 12 atomes de carbone.

4. Composé selon l'une quelconque des revendications 1 à 3, de formule (I-3) suivante :

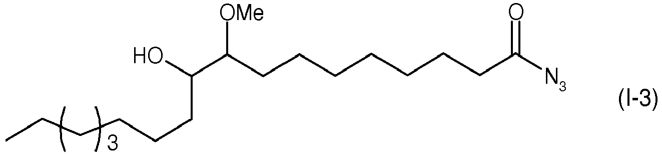

5. Composé selon la revendication 1, de formule (I-5) suivante : 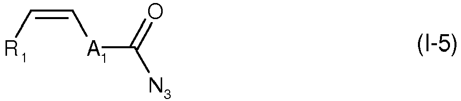 dans laquelle :
- R₁ représente un groupe alkyle, linéaire ou ramifié, comprenant de 6 à 12 atomes de carbone, substitué par un groupe OH,
- A₁ représente un radical alkylène divalent, linéaire ou ramifié, comprenant de 6 à 12 atomes de carbone.

6. Composé selon la revendication 5, de formule (I-7) suivante :

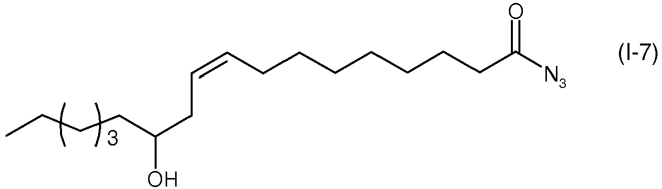

7. Procédé de préparation d'un composé de formule (I) selon l'une quelconque des revendications 1 à 6, comprenant une étape de réaction d'un composé de formule (II) suivante : 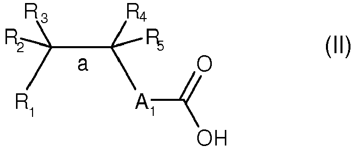 dans laquelle R₁, R₂, R₃, R₄, R₅, A₁ et a sont tels que définis dans la revendication 1,
avec du chloroformate d'éthyle en présence de triéthylamine, puis avec de l'azoture de sodium.

8. Utilisation d'un composé de formule (I) selon l'une quelconque des revendications 1 à 6, pour la préparation de polyuréthane.

9. Utilisation d'un composé de formule (I) selon l'une quelconque des revendications 3 ou 4, pour la préparation de polyuréthane répondant à la formule (III-1) suivante : 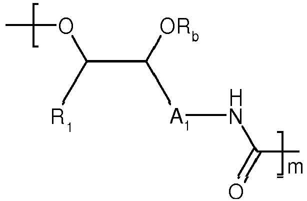 A₁, R₁ et R_{b} étant tels que définis dans la revendication 4, et
m représentant un nombre entier compris de 2 à 50 000.

10. Utilisation d'un composé de formule (I) selon l'une quelconque des revendications 5 ou 6, pour la préparation de polyuréthane répondant à la formule (III-2) suivante : 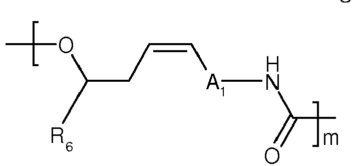 A₁ étant tel que défini dans la revendication 6, R₆ représentant un groupe alkyle, linéaire ou ramifié, comprenant de 1 à 12 atomes de carbone, et m représentant un nombre entier compris de 2 à 50 000.

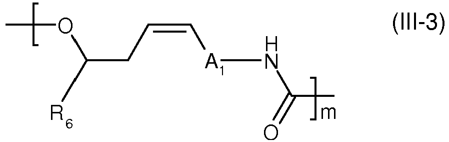

11. Procédé de préparation de polyuréthane, comprenant une étape d'auto-condensation d'un composé selon l'une quelconque des revendications 1 à 6 à une température comprise de 50 ° C à 100°C pendant une durée de 1 heure à 48 heures.

12. Procédé de préparation de polyuréthane, comprenant les étapes suivantes :
- une étape de réaction d'un composé selon l'une quelconque des revendications 1 à 6, avec un alcool R₇OH, R₇ représentant un groupe alkyle, linéaire ou ramifié, comprenant de 1 à 4 atomes de carbone, pour obtenir un composé de formule (IV) suivante : 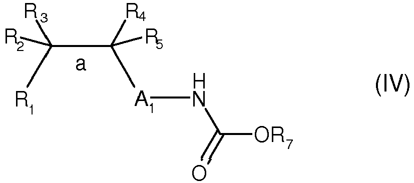 dans laquelle R₁, R₂, R₃, R₄, R₅, A₁ et a sont tels que définis dans la revendication 1, et une étape de polycondensation du composé de formule (IV) en présence de tétrabutoxyde de titane à une température comprise de 80°C à 180°C, pendant une durée de 2 heures à 48 heures.

## Patentansprüche

1. Verbindung der folgenden Formel (I): 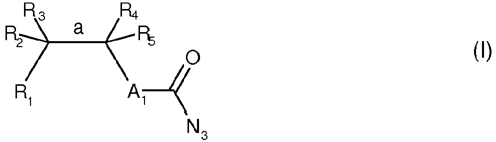 wobei:
- R₁ eine lineare oder verzweigte Alkylgruppe umfassend von 1 bis 20 Kohlenstoffatome darstellt, gegebenenfalls substituiert mit einem oder mehreren Substituenten ORₐ, wobei Rₐ ein Wasserstoffatom oder eine Alkyl-Gruppe umfassend von 1 bis 20 Kohlenstoffatome darstellt, wobei die Gruppe R₁ eventuell eine oder mehrere ungesättigte Bindungen enthalten kann, oder R₁ ein mono-, bi- oder tricyclisches, gesättigtes oder teilweise ungesättigtes, nichtaromatisches Cycloalkyl-Radikal umfassend von 3 bis 20 Kohlenstoffatome darstellt;
- a eine Einfach- oder Doppelbindung darstellt;
- R₂ ein Wasserstoffatom, eine Gruppe ORₐ, wobei Rₐ ein Wasserstoffatom oder eine Alkylgruppe umfassend von 1 bis 20 Kohlenstoffatome darstellt, oder eine lineare oder verzweigte Alkyl-Gruppe umfassend 1 bis 20 Kohlenstoffatome, substituiert mit einer OH-Gruppe, darstellt,
oder R₂ ein Radikal der Formel -(OCH₂CH₂)ₙ-OH oder -CH₂-(CH₂OCH₂)ₙ-CH₃ darstellt, wobei n eine ganze Zahl von 1 bis 100, vorzugsweise von 6 bis 50 und bevorzugt gleich 6, 13 oder 45 darstellt;
- R₃, wenn a eine Einfachbindung ist, ein Wasserstoffatom darstellt, oder, wenn a eine Doppelbindung ist, R₃ abwesend ist;
- R₄ ein Wasserstoffatom oder eine Gruppe R_{b}, wobei R_{b} ein Wasserstoffatom oder eine Alkylgruppe umfassend von 1 bis 20 Kohlenstoffatome darstellt, oder eine lineare oder verzweigte Alkyl-Gruppe umfassend von 1 bis 20 Kohlenstoffatome, substituiert mit einer OH-Gruppe, darstellt,
oder R₄ ein Radikal der Formel -(OCH₂CH₂)ₙ-OH oder -CH₂-(CH₂OCH₂)ₙ-CH₃ darstellt, wobei n eine ganze Zahl von 1 bis 100, vorzugsweise von 6 bis 50 und bevorzugt gleich 6, 13 oder 45 darstellt;
- R₅, wenn a eine Einfachbindung ist, ein Wasserstoffatom darstellt, oder, wenn a eine Doppelbindung ist, R₅ abwesend ist; und
- A₁ ein bivalentes, lineares oder verzweigtes Alkylen-Radikal umfassend von 1 bis 20 Kohlenstoffatome darstellt, wobei das Alkylen-Radikal eventuell eine oder mehrere ungesättigte Bindungen enthalten kann,
wobei mindestens eine der Gruppen R₁, R₂ und R₄ eine OH- oder Alkoxy-Gruppe umfasst.

2. Verbindung gemäß Anspruch 1, der Formel (I), wobei, wenn a eine Einfachbindung ist, mindestens eine der Gruppen R₂ und R₄ eine OH- oder AlkoxyGruppe umfasst.

3. Verbindung gemäß einem der Ansprüche 1 oder 2, der folgenden Formel (1-2): 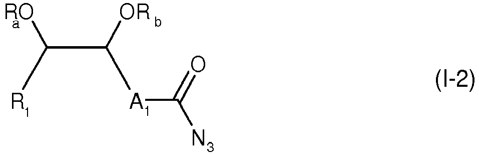 wobei:
- R₁ eine lineare oder verzweigte Alkyl-Gruppe umfassend von 6 bis 12 Kohlenstoffatome darstellt,
- Rₐ ein Wasserstoffatom oder eine Alkyl-Gruppe umfassend von 1 bis 12 Kohlenstoffatome darstellt,
- R_{b} ein Wasserstoffatom oder eine Alkyl-Gruppe umfassend von 1 bis 12 Kohlenstoffatome darstellt,
- A₁ ein bivalentes, lineares oder verzweigtes Alkylen-Radikal umfassend von 6 bis 12 Kohlenstoffatome darstellt.

4. Verbindung gemäß einem der Ansprüche 1 bis 3, der folgenden Formel (1-3):

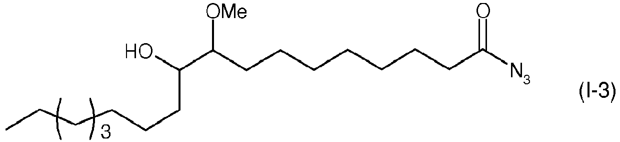

5. Verbindung gemäß Anspruch 1, der folgenden Formel (1-5): 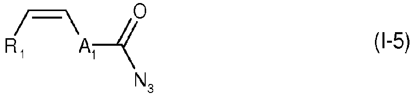 wobei:
- R₁ eine lineare oder verzweigte Alkyl-Gruppe, umfassend von 6 bis 12 Kohlenstoffatome, substituiert mit einer OH-Gruppe, darstellt,
- A₁ ein bivalentes, lineares oder verzweigtes Alkylen-Radikal umfassend von 6 bis 12 Kohlenstoffatome darstellt.

6. Verbindung gemäß Anspruch 5, der folgenden Formel (1-7):

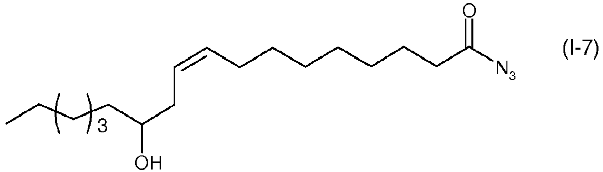

7. Verfahren zur Herstellung einer Verbindung der Formel (I) gemäß einem der Ansprüche 1 bis 6, umfassend einen Schritt des Umsetzens einer Verbindung der folgenden Formel (II): 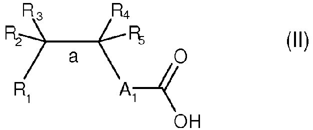 wobei R₁, R₂, R₃, R₄, R₅, A₁ und a wie in Anspruch 1 definiert sind,
mit Ethylchloroformat in Anwesenheit von Triethylamin, hiernach mit Natriumazid.

8. Verwendung einer Verbindung der Formel (I) gemäß einem der Ansprüche 1 bis 6 für die Herstellung von Polyurethan.

9. Verwendung einer Verbindung der Formel (I) gemäß einem der Ansprüche 3 oder 4 für die Herstellung von Polyurethan entsprechend der folgenden Formel (III-1): 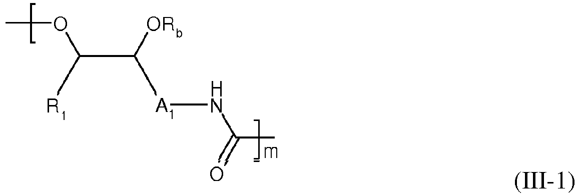 wobei A₁, R₁ und R_{b} wie in Anspruch 4 definiert sind, und
m eine ganze Zahl von 2 und 50000 darstellt.

10. Verwendung einer Verbindung der Formel (I) gemäß einem der Ansprüche 5 oder 6 für die Herstellung von Polyurethan entsprechend der folgenden Formel (III-2): 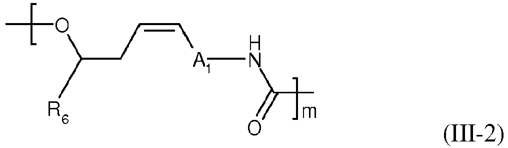 wobei A₁ wie in Anspruch 6 definiert ist, R₆ eine lineare oder verzweigte Alkyl-Gruppe umfassend von 1 bis 12 Kohlenstoffatome darstellt und m eine ganze Zahl von 2 und 50000 darstellt.

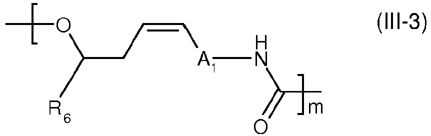

11. Verfahren zur Herstellung von Polyurethan, umfassend einen Schritt der Autokondensation einer Verbindung gemäß einem der Ansprüche 1 bis 6 bei einer Temperatur zwischen 50 °C und 100 °C über eine Dauer von 1 Stunde bis 48 Stunden.

12. Verfahren zur Herstellung von Polyurethan, umfassend die folgenden Schritte:
- einen Schritt des Umsetzens einer Verbindung gemäß einem der Ansprüche 1 bis 6 mit einem Alkohol R₇OH, wobei R₇ eine lineare oder verzweigte Alkyl-Gruppe umfassend von 1 bis 4 Kohlenstoffe darstellt, um eine Verbindung der folgenden Formel (IV) zu erhalten: 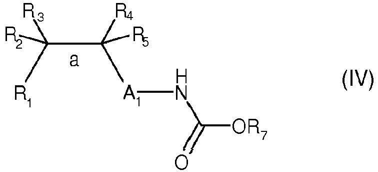 wobei R₁, R₂, R₃, R₄, R₅, A₁ und a wie in Anspruch 1 definiert sind, und
einen Schritt der Polykondensation der Verbindung der Formel (IV) in Anwesenheit von Titantetrabutoxid bei einer Temperatur von 80 °C bis 180 °C, über eine Dauer von 2 Stunden bis 48 Stunden.

## Claims

1. A compound of following formula (I): 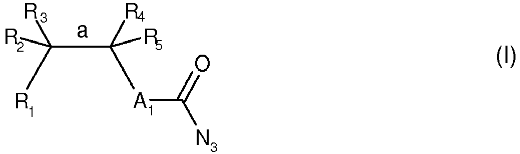 in which:
- R₁ is a straight-chain or branched alkyl group comprising 1 to 20 carbon atoms, optionally substituted by one or more ORₐ substituents, Rₐ representing a hydrogen atom or an alkyl group comprising 1 to 20 carbon atoms, the said group R₁ optionally containing one or more unsaturations; or R₁ represents a mono-, bi- or tri- cyclic hydrocarbon radical, saturated or partly unsaturated, non-aromatic, comprising from 3 to 20 carbon atoms;
- a represents a single or double bond;
- R₂ is a hydrogen atom, an ORₐ group, Rₐ representing a hydrogen atom or an alkyl group comprising 1 to 20 carbon atoms, or a straight-chain or branched alkyl group comprising 1 to 20 carbon atoms substituted by an OH group,
or R₂ is a radical of formula -(OCH₂CH₂)ₙ-OH or -CH₂-(CH₂OCH₂)ₙ-CH₃, n representing an integer of 1 to 100, preferably 6 to 50, preferably it is 6, 13 or 45;
- R₃, when a is single bond, represents a hydrogen atom or, when a is a double bond, R₃ is absent;
- R₄ is a hydrogen atom or an OR_{b} group, R_{b} representing a hydrogen atom or an alkyl group comprising 1 to 20 carbon atoms, or a straight-chain or branched alkyl group comprising 1 to 20 carbon atoms substituted by an OH group,
or R₄ is a radical of formula -(OCH₂CH₂)ₙ-OH or -CH₂-(CH₂OCH₂)ₙ-CH₃, n representing an integer of 1 to 100, preferably 6 to 50, and preferably it is 6, 13 or 45;
- R₅, when a is a single bond, represents a hydrogen atom, or when a is a double bond R₅ is absent; and
- A₁ is a divalent alkylene radical, straight-chain or branched, comprising 1 to 20 carbon atoms, the said alkylene radical optionally containing one or more unsaturations, and
wherein at least one of the groups R₁, R₂ and R₄ comprises an OH or alkoxy group.

2. The compound according₂
to claim 1 of formula (I) wherein, when a represents a single bond, at least one of the groups R₂ and R₄ comprises an OH or alkoxy group.

3. The compound according to any of claims 1 to 2 of the following formula (I-2): 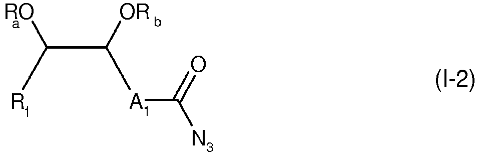 where:
- R₁ is a straight-chain or branched alkyl group comprising 6 to 12 carbon atoms,
- Rₐ is a hydrogen atom or an alkyl group comprising 1 to 12 carbon atoms,
- R_{b} is a hydrogen atom or an alkyl group comprising 1 to 12 carbon atoms, and
- A₁ is a divalent alkylene radical, straight-chain or branched, comprising 6 to 12 carbon atoms.

4. The compound according to any of claims 1 to 3 of the following formula (I-3):

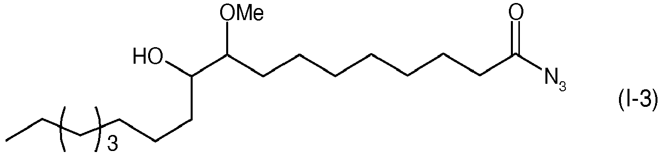

5. The compound according to claim 1 of the following formula (I-5): 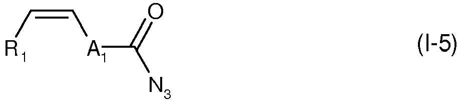 where:
- R₁ is a straight-chain or branched alkyl group comprising 6 to 12 carbon atoms, substituted by an OH group,
- A₁ is a divalent alkylene radical, straight-chain or branched, comprising 6 to 12 carbon atoms.

6. The compound according to claim 5 of the following formula (I-7):

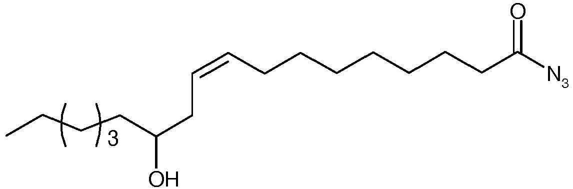

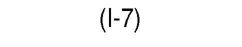

7. A method for preparing a compound of formula (I) according to any of claims 1 to 6, comprising a reaction step of a compound of the following formula (II): 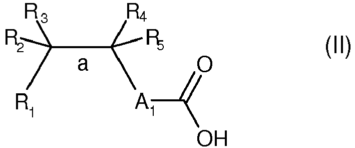 where R₁, R₂, R₃, R₄, R₅, A₁ and a, are such as defined in claim 1,
with ethyl chloroformate in the presence of triethylamine, then with sodium azide.

8. The use of a compound of formula (I) according to any of claims 1 to 6 for the preparation of polyurethane.

9. The use of a formula (I) compound according to any of claims 3 or 4 for the preparation of polyurethane meeting the following formula (III-I) : 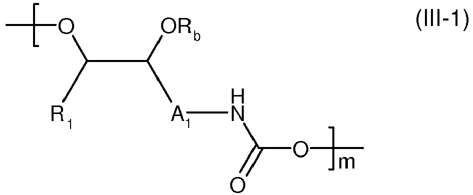 A₁, R₁ and R_{b} being such as defined in claim 4, and
m representing an integer of 2 to 50 000.

10. The use of a formula (I) compound according to any of claims 5 or 6 for the preparation of polyurethane meeting the following formula (III-2): 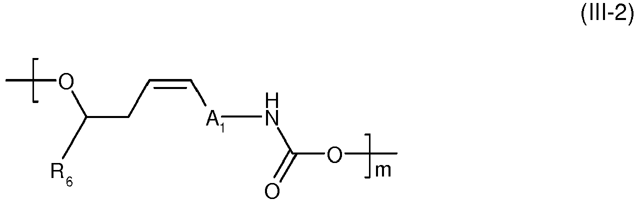 A₁ being such as defined in claim 6, R₆ representing a straight-chain or branched alkyl group comprising 1 to 12 carbon atoms, and m representing an integer of 2 to 50 000.

11. A method for preparing polyurethane, comprising a self-condensation step of a compound according to any of claims 1 to 6 at a temperature of between 50°C to 100°C, for a time of 1 hour to 48 hours.

12. A method for preparing polyurethane, comprising the following steps :
- a reaction step of a compound according to any of claims 1 to 6 with an alcohol R₇OH, R₇ representing a straight-chain or branched alkyl group comprising 1 to 4 carbon atoms, to obtain a compound of the following formula (IV): 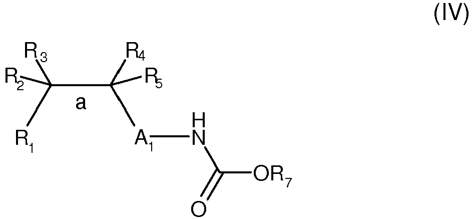 where R₁, R₂, R₃, R₄, R₅, A₁ and a, are such as defined in claim 1, and
- a polycondensation step of the formula (IV) compound in the presence of titanium tetrabutoxide at a temperature of between 80°C and 180°C, for a time of 2 hours to 48 hours.
